(19) **Europäisches Patentamt**

**European Patent Office**

**Office européen des brevets**

(11) **EP 4 682 828 A1**

(12) **EUROPEAN PATENT APPLICATION**

(43) Date of publication:
21.01.2026 Bulletin 2026/04

(21) Application number: 24189244.7

(22) Date of filing: 17.07.2024

(51) International Patent Classification (IPC):
**G06T 11/00** $^{(2026.01)}$   **A61B 6/00** $^{(2024.01)}$

(52) Cooperative Patent Classification (CPC):
**G06T 11/005; A61B 6/032; A61B 6/4241;**
A61B 6/482; G06T 2207/10081; G06T 2207/10116;
G06T 2211/408

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Koninklijke Philips N.V.**
**5656 AG Eindhoven (NL)**

(72) Inventors:
• **BRENDEL, Bernhard Johannes**
**Eindhoven (NL)**
• **SOSSIN, Artur**
**Eindhoven (NL)**
• **DAERR, Heiner**
**5656AG Eindhoven (NL)**
• **THRAN, Axel**
**Eindhoven (NL)**

(74) Representative: **Philips Intellectual Property & Standards**
**High Tech Campus 52**
**5656 AG Eindhoven (NL)**

(54) **OFF-FOCAL CORRECTION FOR SPECTRAL X-RAY IMAGING**

(57) A method for off-focal correction applied to x-ray measurement data, e.g. to CT projection data. The method is based on performing a first pass material decomposition to derive material decomposition data for two or more materials in the imaged body followed by performing a virtual attenuation of a known emission spectrum of the off-focal radiation along an estimated material path from the off-focal emission area of the x-ray source to each pixel of the detector using the material decomposition data derived in the first pass.

EP 4 682 828 A1

FIG. 1

**Description**

FIELD OF THE INVENTION

The present invention relates to a method for pre-processing of x-ray data for improved image reconstruction, in particular a method for off-focal radiation correction.

BACKGROUND OF THE INVENTION

Off-focal correction is an artefact reduction technique used in x-ray imaging, e.g. computed tomography (CT) imaging and DXR imaging to improve image quality.

In CT scanning, x-rays are emitted from a source, which is typically a focal spot on an anode of an x-ray rube. The x-rays pass through a body being scanned to create images based on detection of the attenuated radiation on an opposite side of the body. However, not all emitted x-ray radiation originates from the focal spot; some is emitted from areas around the focal spot. This is known as off-focal radiation. The same effect can occur in any other type of x-ray based imaging which uses an x-ray tube source.

Off-focal radiation can create artefacts in the reconstructed images. The artifacts are especially noticeable at high contrast edges of an object. High contrast edges of an object are boundaries between media where there is a particularly sharp transition between different types of tissue or between tissue and air.

High contrast edges are common in the human body. One example is a boundary between bone and soft tissue. In head-imaging, off-focal radiation leads to bright artifacts in the brain near the skull.

The resulting artifacts have the effect of making the edges appear blurred or with streaks. This can obscure true anatomical details and potentially lead to misinterpretation of the image.

Off-focal correction is a technique that may be applied during the pre-processing of the x-ray data. In the context of CT imaging, pre-processing typically means processing applied to the acquired CT projection data before reconstruction of the final image.

Conventionally, off-focal correction is achieved through de-convolution operations or high-pass filtering applied to the collected projection data. These methods mathematically refine the raw data to reduce the distortion caused by off-focal radiation.

Advanced x-ray tubes are available which operate with unipolar acceleration voltage and have the anode at ground potential, and are equipped with a back-scattered electron capture unit. The electron capture unit has the effect of minimizing the number of electrons which contribute to off-focal radiation. These tubes are able to reduce off-focal radiation to some extent but not fully.

Less advanced x-ray tubes are available which use a bipolar acceleration voltage and lack an electron capture unit. These do not control for back-scattered electrons as effectively. As a result, these electrons can be re-accelerated towards the anode, leading to increased off-focal radiation.

It would be of advantage to provide a solution to more effectively reduce off-focal artefacts using a software-based approach, thereby improving image quality in a way that does not necessitate complex and expensive hardware adaptations.

SUMMARY OF THE INVENTION

The invention is defined by the claims.

According to examples in accordance with an aspect of the invention, there is provided a method for off-focal correction of spectral x-ray data acquired using a spectral x-ray imaging apparatus comprising an x-ray source which emits primary radiation from a focal spot area of the x-ray source and off-focal radiation from a second area of the x-ray source outside of the focal spot area.

The method comprises receiving spectral x-ray measurement data comprising measurement data for each of a plurality of pixels of an x-ray detector in each of two or more energy channels for at least one x-ray projection.

The method further comprises applying a material decomposition algorithm to convert the spectral measurement data into material measurement data, where the material measurement data comprises, for each pixel of the detector, a respective attenuation contribution of each of two or more materials along a beam path from the x-ray source to the detector pixel.

The method may further comprise obtaining an indication of an x-ray absorption spectrum for each of the two or more materials.

The method may further comprise obtaining an indication of an x-ray emission spectrum for the off-focal radiation emitted by the x-ray source.

The method may further comprise, for each pixel of the measurement data of the at least one projection, using the material measurement data to compute an estimated attenuation of the said off-focal radiation, having the said emission spectrum, by the said two or more materials, having the said x-ray absorption spectra, along an expected beam path from the second area of the x-ray source to the detector pixel to thereby compute an estimated off-focal radiation contribution to the spectral x-ray measurement data of the pixel, in each spectral channel.

The method may further comprise correcting the received spectral measurement data to remove or reduce the estimated off-focal contribution for each spectral channel for each pixel of the measurement data to yield corrected spectral x-ray measurement data.

Thus, embodiments of the invention are based on the principle of applying a first pass material decomposition to acquired projection data to obtain initial material decomposition data and then using the initial material decomposition data to estimate an off-focal radiation contribution to the projection data of each pixel. This is done by using the estimated radiation contribution along the estimated beam path from the off-focal source area(s) to the given pixel, these being extracted from the initial material decomposition. For example, the material measurement data may comprise, for each pixel, and for each projection, computed material path lengths along the beam trajectory from the x-ray source to the respective pixel and among these a beam trajectory from source to pixel can be found which as closely as possible matches the expected beam path from the off-focal (second) area of the x-ray source to a given pixel whose data is to be corrected. By applying the forward model along this path, using the material path lengths computed for it, and using the emission spectrum for the off-focal radiation, an estimate can be made of the expected off-focal radiation reaching the pixel to-be-corrected after attenuation by the materials lying along that path. The most closely matching beam path can be found among the measurement data for other pixels of the same projection or the same or other pixels of a different projection (i.e. once the detector/source pair have rotated to a new position).

By way of example, for each pixel of the measurement data, the method may comprise:

- determining an estimated beam path/trajectory from the second area of the x-ray source to the corresponding pixel of the detector; and
- estimating from the material decomposition data an estimated attenuation of the off-focal radiation, having the said off-focal radiation absorption spectrum, by each of the two or more materials, along said estimated beam path.

In some embodiments, the method further comprises generating a data output representative of the corrected spectral measurement data. The method may further comprise communicating the data output to a further computing device.

The obtaining of the x-ray absorption spectrum for each of the two or more materials may comprise retrieving a record of the x-ray absorption spectrum of each of the two or more materials from a memory. The obtaining the x-ray emission spectrum for the off-focal radiation may comprise retrieving a record of the x-ray emission spectrum from a memory.

In some embodiments, the method further comprises re-computing a material decomposition using the corrected spectral measurement data.

In some embodiments, the step of determining the respective attenuation contribution of each of the two or more materials along a beam path from the second area of the source to the detector pixel comprises: determining, from the material measurement data, an estimated material path length of each of the two or more materials along the expected beam path from the second area of the source to the detector pixel.

In some embodiments, the step of computing an estimated attenuation of the off-focal emission spectrum along an expected beam path from the second area of the x-ray source to a detector pixel further comprises: computing an estimated attenuation of the off-focal radiation along a path comprising or characterized by said determined material path lengths.

To explain further: the computed material measurement data includes material measurement values for each pixel for each projection, where the set of material measurement values for each pixel typically represent values of material path lengths for the two or more materials along a beam path from the source to the respective pixel. Thus, there can be identified among the measurement data (for the same projection or another projection) material measurement data for another pixel which corresponds to values for material path lengths along a beam path from the source to the detector which matches as closely as possible to the path from the pixel-to-be-corrected and the second area of the x-ray source.

Thus, in some embodiments, for at least a subset of the pixels, the step of determining the material path lengths along the expected beam path comprises identifying in the material measurement data computed material path lengths computed for a beam path from the focal spot area of the x-ray source to a different detector pixel of a same x-ray projection.

This beam path for the different pixel may be a beam path which is determined to be sufficiently close in trajectory to the expected beam path from the second area of the x-ray source to the pixel-to-be-corrected. For example, the beam path may be a beam path which is determined as meeting a pre-defined similarity criterion relating to a degree of similarity with the expected beam path from the second area of the x-ray source to the pixel-to-be-corrected.

For example, the beam path may be a beam path having a path trajectory which is within a threshold degree of alignment spatially with said expected beam path.

In further embodiments, for at least a subset of the pixels, the step of determining the material path lengths along the expected beam path comprises identifying in the material measurement data computed material path lengths computed for a beam path from the focal spot area of the x-ray source to a same or different detector pixel of a different x-ray projection.

Again, this beam path for the different pixel may be a beam path which is determined to be sufficiently close in trajectory to the expected beam path from the second area of the x-ray source to the pixel-to-be-corrected. For example, the beam path may be a beam path which is determined as meeting a pre-defined similarity criterion relating to a degree of similarity with the expected beam path from the second area of the x-ray source to the pixel-to-be-corrected.

A single projection in this context means a single projection from the source to the detector, resulting in a single set of spectral measurement/detection data values at each pixel of the detector, for example a single measurement value for each energy bin/level for each pixel of the detector. A single projection for example is a set of measurement/detection data at each pixel and in each energy level/bin for a single given rotational and axial position of the x-ray detector and x-ray source pair.

In some embodiments, the x-ray imaging apparatus is a computed tomography (CT) imaging apparatus, and wherein the x-ray measurement data is CT projection data acquired by the CT imaging apparatus.

Another aspect of the invention is a computer program product comprising computer program code configured, when run on a processor, to cause the processor to perform a method in accordance with any preceding claim.

Another aspect of the invention is a processing device comprising one or more processors configured to perform a method in accordance with any embodiment or example described in this disclosure, or in accordance with any claim.

Another aspect of the invention is a system, comprising a processing device in accordance with any embodiment or example described in this disclosure, or in accordance with any claim; and an x-ray imaging apparatus operatively coupled with the processing device.

In some embodiments, the x-ray imaging apparatus is a computed tomography (CT) imaging apparatus operatively coupled with the processing device.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Fig. 1 outlines steps of an example method in accordance with one or more embodiments of the invention;
Fig. 2 is a block diagram of an example processing device and system in accordance with one or more embodiments of the invention;
Fig. 3 schematically illustrates an example CT imaging apparatus suitable for use with one or more embodiments of the invention;
Fig. 4 schematically illustrates spectral measurement data for an example projection acquired in a CT scan;
Fig. 5 schematically illustrates off-focal radiation generated by an x-ray tube during a projection event;
Figs. 6-8 schematically illustrates emission of off-focal radiation from an x-ray source toward a detector;
Fig. 9 schematically illustrates the concept of inferring an attenuation contribution of off-focal radiation at a given detector pixel based on utilizing material decomposition data of one or more neighboring detector pixels;
Fig. 10 schematically illustrates angular projection ranges of radiation emitted from a focal spot area of the x-ray source to each of a row of pixels of an x-ray detector; and
Fig. 11 schematically illustrates an approach for selection of one or more neighboring pixels whose data is to be used for estimating an off-focal contribution to a pixel-to-be-corrected.

DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a method for off-focal correction applied to x-ray measurement data, e.g. to CT projection data. The method is based on performing a first pass material decomposition operation to derive material decomposition data for two or more materials in the imaged body followed by performing a virtual attenuation of a known emission spectrum of the off-focal radiation along an estimated material path from the off-focal emission area of the x-ray source to each pixel of the detector using the material decomposition data derived in the first pass.

For example, the overall material decomposition data includes material path lengths for each detector pixel and for each x-ray projection, these path lengths corresponding to different path trajectories through the imaging zone (depending upon the specific pixel location and the source/detector locations). From these, an estimated material path substantially corresponding in trajectory to the expected path from the off-focal area of the source and a given pixel can be found, and an attenuation of a known off-focal radiation spectrum along the material path calculated. This can be used to provide an estimate of the off-focal radiation contribution to the relevant pixel, for example by applying the forward model to estimate the received radiation from the off-focal source at the pixel-to-be-corrected after passing along the identified material path having the material path lengths.

By way of further summary, to mitigate the quantification errors which arise in conventional off-focal correction, embodiments of the present invention propose a procedure for spectral off-focal correction that considers the different spectra of off-focal radiation and primary radiation.

In accordance with at least one set of embodiments, the basic steps of the proposed method may include at least one or more of the following:

1. Performing a first material decomposition neglecting the off-focal correction;
2. Performing a procedure to estimate from the first material decomposition the contribution of off-focal radiation to the measurement of each detector pixel;
3. Performing a correction of the spectral measurements that removes the off-focal contribution;
4. Optionally further performing a second material decomposition on the corrected spectral measurements.

Finally material or spectral images may be reconstructed from the output of the last material decomposition.

Optionally, steps 2-4 may be repeated one or more times, e.g. executed iteratively, to improve the accuracy of the off-focal correction.

Embodiments of the present invention are applicable to any source of spectral x-ray data. One advantageous application is for spectral CT projection data. By way of background, brief explanation of the basic principles of material decomposition as applied in spectral x-ray based imaging (including but not limited to CT imaging) will now be discussed.

By using multi-energy x-ray detectors, and/or a multi-energy x-ray source, it is possible to measure the spectrum of x-ray energies or frequencies received at different locations across an x-ray detector during x-ray imaging of an object. In particular, it is possible to measure detection data at each pixel in each of a plurality of different x-ray energy levels or energy bands. This is known as spectral x-ray imaging. Spectral x-ray data allows for discerning and quantifying materials comprised by the scanned object. It can be performed using data from a conventional x-ray imaging modality or from CT imaging.

Material decomposition can be achieved based on a pixel-wise inversion of the system forward model, which model describes the theoretical signal expected in individual x-ray detector energy bins as a function of a certain set (basis) of materials comprised by the object and their respective equivalent path lengths along the line from the focal spot to the detector pixel. This procedure is called basis material decomposition.

The basic principles of spectral x-ray based material decomposition, as applied in embodiments of the present invention, will now be briefly discussed. A more detailed discussion of the underlying principles of material decomposition applied herein can also be found in the paper: K. C. Zimmerman and T. G. Schmidt, Experimental comparison of empirical material decomposition methods for spectral CT, Physics in Medicine & Biology, 60 (2015) 3175-3191, doi:10.1088/0031-9155/60/8/3175.

When an x-ray photon strikes a photon-counting detector, the photon is converted to electrical charge proportional to the energy of the incoming photon. The charge may be converted to a voltage using charge-integrating amplifiers. Analog comparators can be used to increment a digital counter when the voltage of the accumulated charge exceeds a set threshold level. At the end of an acquisition, the number of photons detected with energy above the threshold can be counted. Energy bin data can be generated corresponding to the number of photons detected between two threshold levels based on subtracting consecutive counter measurements. In this way, a photon count for a plurality of x-ray energy bins can be generated.

It is noted that for the purpose of the following description, use of a spectral photon counting detector will be assumed. However, other types of detector are also possible such as spectral energy integrating detectors (EIDs) which are able to detect radiation within a particular window of the energy spectrum of the X-ray source.

The case may be considered of an x-ray measurement through a material of thickness x and attenuation coefficient $\mu(E)$, where E is the energy of photon traversing the material. The x-ray attenuation of a composite material can be expressed as a linear combination of the attenuation of a set of basis materials comprised by the material. More particularly, and as explained in K. C. Zimmerman (2015), the x-ray attenuation through this material is equivalent to the attenuation of a unique combination of any two other materials (in the absence of K-edges), as expressed in equation (1) below, where $\mu_1(E)$ and $\mu_2(E)$ are the energy-dependent attenuation coefficients of each of two basis materials and $l_1$ and $l_2$ are the path lengths of each of the two basis materials. This decomposition is possible because there exist two primary attenuation phenomena in the diagnostic x-ray energy range: Compton scattering and photoelectric absorption.

$$x\mu(E) = l_1\mu_1(E) + l_2\mu_2(E) \tag{1}$$

This basis expansion can be extended, so that the expected number of photons detected in the $i^{th}$ energy bin, $c_i$, of an ideal photon-counting detector can be modelled as,

$$c_i(\boldsymbol{l}) = \int_{E_i}^{E_{i+1}} S(E)\exp\left[-\sum_{j=1}^{M} l_j\mu_j(E)\right]dE \tag{2}$$

where $S(E)$ is the x-ray source spectrum and $l_j$, the elements of $l$, are the thicknesses of M basis materials having attenuation coefficients functions, $\mu_j$. The spectral measurements are represented as a vector of detected photon counts, $\mathbf{c} = [c_1, c_2,..., c_K]^T$, where K is total the number of energy measurements. It is noted that Equation 2 above represents the case for an ideal photon-counting detector and does not take detector sensitivity into account.

Material decomposition in general terms involves estimating the basis material thicknesses or path lengths, $l$, from the acquired spectral data, c. One method of estimating the basis material thicknesses, $l$, from the number of detected photons, $\mathbf{c}$, is to numerically invert equation (2), for example using statistical estimation algorithms such as maximum likelihood estimation, (MLE).

Another approach which is often employed is to use a simple look-up table which allows for mapping between detection data and material path lengths. Other approaches are also possible including applying a polynomial model, or implementing an artificial intelligence (AI)-based model.

In the context of material decomposition within spectral x-ray imaging, "path length" refers to the physical distance that x-ray photons travel through a specific material within the subject being imaged along a beam path from the source to the relevant detector pixel. Each type of material in the subject's body - such as bone, soft tissue, or contrast agents - has a unique attenuation coefficient, $\mu$, which determines how much the x-ray beam is absorbed or scattered while passing through it. In the context of the above description, the vector $l$ represents the vector of material path lengths for the different basis materials.

In the context of this disclosure, the term 'x-ray measurement data' refers to raw measurement data acquired by the x-ray detector, which includes measurements for example for two or more different energy levels/channels/bins at each pixel of the detector array and for each x-ray projection. In the context of CT imaging, this is sometime referred to as spectral projection data. A single projection in this context means a single projection from the source to the detector, resulting in a single set of spectral measurement/detection data values at each pixel of the detector, for example a single measurement value for each energy bin/level for each pixel of the detector. A single projection for example is a set of measurement/detection data at each pixel and in each energy level/bin for a single given rotational and axial position of the x-ray detector and x-ray source pair. A single projection could be acquired through a single multi-energy x-ray emission event from the x-ray source which is detected by an energy discriminating detector, or it could be acquired via multiple emission events from the source at a sequence of two or more different x-ray energies, each being detected by the detector and the set of detections forming the spectral (multi-energy) measurement data for the respective projection.

In the context of this disclosure, 'material measurement data' refers to the output of the material decomposition procedure applied to the x-ray measurement data but before any application of image reconstruction. It is sometime called material-specific measurement data. In the case of CT imaging, this is sometimes referred to as material projection data. In some examples, this may take the form of material path lengths, $l$, for each basis material, for each pixel of the detector and for each x-ray projection.

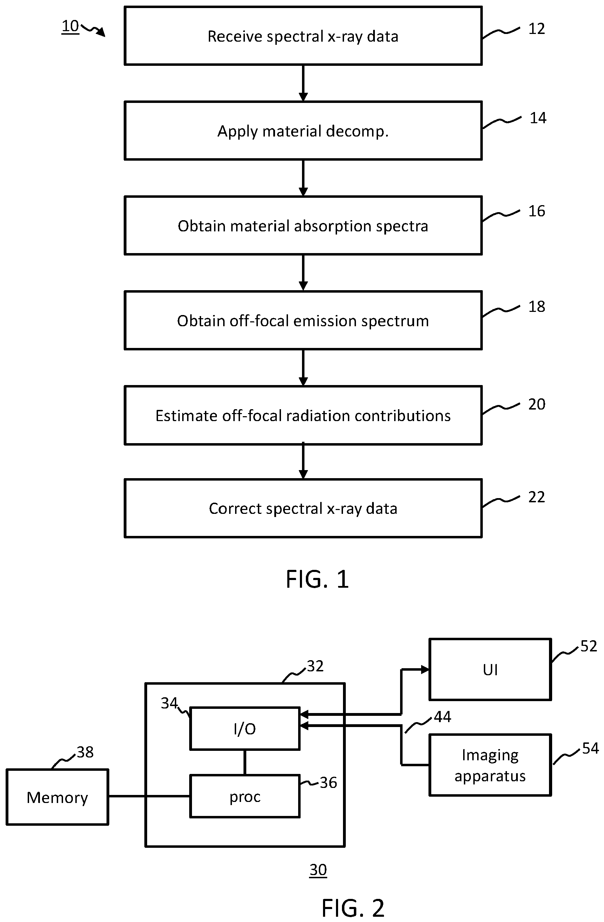

Fig. 1 outlines in block diagram form steps of an example method according to one or more embodiments. The steps will be recited in summary, before being explained further in the form of example embodiments.

The provided method is for off-focal correction of spectral x-ray data acquired using a spectral x-ray imaging apparatus. The off-focal correction is for example for reducing a contribution of off-focal radiation emitted by an x-ray source of the x-ray imaging apparatus. The x-ray source for example emits primary radiation from a focal spot area of the x-ray source and off-focal radiation from a second area of the x-ray source outside of the focal spot area.

The method 10 comprises receiving 12 spectral x-ray measurement data. The spectral x-ray measurement data may comprise detector data from a multi-energy x-ray detector (e.g. a spectrally resolving x-ray detector). The detector data may include measurement data for each of a plurality of pixels of the x-ray detector in each of two or more energy channels for at least one x-ray projection. Where the x-ray imaging apparatus is a CT imaging apparatus, the x-ray measurement data may be referred to as projection data. The two or more different energy channels correspond to detection of radiation at two or more different energy levels, e.g. energy ranges.

The method 10 further comprises applying 14 a (first) material decomposition algorithm to convert the spectral measurement data into material measurement data. The material measurement data may comprise, for each pixel of the detector, a respective attenuation contribution of each of two or more materials along a beam path from the focal spot area of the source to the detector pixel. The attenuation contribution may be an attenuation path length, otherwise known as a material path length. A material path length means an estimated distance or thickness of each of the two or more materials lying along the x-ray beam path between the x-ray source and the detector and which therefore contribute to the attenuation of the x-ray beam along that path. In the context of CT imaging, the material path lengths are sometimes referred to as material line integrals.

The method 10 further comprises obtaining or receiving 16 (e.g. from a memory or database or register) an indication of an x-ray absorption coefficient for each of the two or more materials for at least each of the two or more energy levels corresponding to the energy channels of the measurement data. For example, this may comprise receiving an indication of an absorption spectrum for each of the two or more materials.

The method may further comprise obtaining or receiving 18 (e.g. from a memory or database or register) an indication of an x-ray emission spectrum for the off-focal radiation emitted by the x-ray source.

The emission spectra of the primary and off-focal radiation can for example be determined using methods described in the following paper: Ali ES, Rogers DW. Quantifying the effect of off-focal radiation on the output of kilovoltage x-ray systems. Med Phys. 2008 Sep;35(9):4149-60. doi: 10.1118/1.2966348. PMID: 18841868.

The method 10 may further comprise, for each detector pixel of the measurement data, computing 20 an estimated off-focal radiation contribution to the spectral x-ray measurement data of the pixel, in each spectral channel. This may be performed based on computing an estimated attenuation of the said off-focal radiation emission spectrum by the said two or more materials having the said x-ray absorption spectra along an expected beam path from the second area of the x-ray source to the detector pixel. This will be explained in more detail to follow.

The method may further comprise correcting 22 the original spectral measurement data to remove or reduce the computed estimated off-focal contribution for each spectral channel of the measurement data to yield corrected spectral x-ray measurement data. Alternatively, the computed contribution may be saved in a data representation and either stored or transferred to a further processing module.

As noted above, the method can also be embodied in hardware form, for example in the form of a processing device which is configured to carry out a method in accordance with any example or embodiment described in this document, or in accordance with any claim of this application.

To further aid understanding, Fig. 2 presents a schematic representation of an example processing device 32 configured to execute a method in accordance with one or more embodiments of the invention. The processing device is shown in the context of a system 30 which comprises the processing device. The processing device alone represents an aspect of the invention. The system 30 is another aspect of the invention. The provided system need not comprise all the illustrated hardware components; it may comprise only a subset of them.

The processing device 32 comprises one or more processors 36 configured to perform a method in accordance with that outlined above, or in accordance with any embodiment described in this document or any claim of this application. In the illustrated example, the processing device further comprises an input/output 34 or communication interface.

In the illustrated example of Fig. 2, the system 30 further comprises a user interface 52. This is optional.

In the illustrated example of Fig. 2, the system 30 further comprises an x-ray based imaging apparatus 54 for acquiring x-ray measurement data such as a CT imaging apparatus for acquiring CT projection data.

The system 30 may further comprise a memory 38 for storing computer program code (i.e. computer-executable code) which is configured for causing the one or more processors 36 of the processing unit 32 to perform the method as outlined above, or in accordance with any embodiment described in this disclosure, or in accordance with any claim.

As mentioned previously, the invention can also be embodied in software form. Thus another aspect of the invention is a computer program product comprising computer program code configured, when run on a processor, to cause the processor to perform a method in accordance with any example or embodiment of the invention described in this document, or in accordance with any claim of this patent application.

To aid in understanding of the principles of the invention, there will now be briefly discussed an example CT imaging apparatus suitable for utilization with embodiments of the present application.

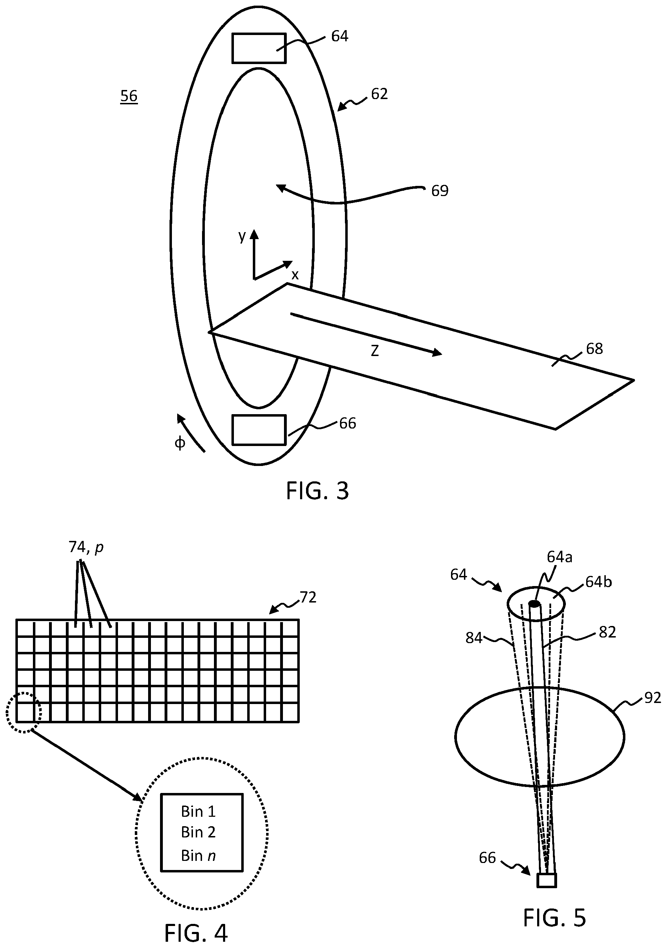

Fig. 3 shows a schematic representation of a CT imaging apparatus 56. The CT imaging apparatus comprises a rotatable gantry 62 housing an x-ray source 64 (e.g. an x-ray tube), and an x-ray array detector 66. The x-ray detector may be a 2D array detector. The CT imaging apparatus further comprises a patient table 68 which is moveable in an axial direction, z, relative to the gantry. With a patient disposed on the table 68, an x-ray beam emitted from the source 64 traverses an imaging zone 69 around which the gantry extends, is attenuated by the tissue of the patient and is detected at the detector 66.

The CT imaging apparatus may be a spectral (or multi-energy) CT imaging apparatus. A spectral CT imaging apparatus generates multi-energy projection data.

With regards to the generation of spectral or multi-energy CT projection data, this can be acquired in different ways. One way is to use a kVp switching x-ray source to alternate an energy level of emitted radiation and to acquire detector measurements for each x-ray source energy for each projection. Another way is to use an energy discriminating detector which is capable of differentially detecting x-ray radiation in two or more different energy bands or levels simultaneously. One example of such an energy discriminating detector is a photon counting detector. Another example of such an energy discriminating detector is an energy integrating detector.

The x-ray source 64 and an x-ray detector 66 are carried at diametrically opposite positions on the rotatable gantry 62. The rotatable gantry extends annularly around an imaging zone 69. The x-ray source is configured to emit a projection of x-ray radiation which passes through the imaging zone and is detected by the x-ray detector on the opposite side of the gantry. The x-ray radiation is attenuated by the materials of any body which is located in the imaging zone 69. The x-ray detector detects an intensity of radiation at each pixel of an array of detector pixels. Based on knowledge of the energy of the x-ray radiation emitted by the source 64, the attenuation by the body can be determined from the detector data. The x-ray source 64 and detector 66 pair are rotatably moveable angularly around the circumference of the gantry 62 in a circumferential/azimuthal direction, $\phi$, for acquiring attenuation data at each of a series of different angular positions, $\phi$. The plane of rotation extends across the x and y dimensions as illustrated in Fig. 3. The gantry 62 is also moveable axially relative a patient table (not shown in Fig. 4) in the z-direction for acquiring attenuation information at different axial positions, z. The axial, z, direction is perpendicular to the x-y plane of rotation. In step-and-shoot acquisition protocols, this relative movement is achieved by moving the patient table while holding the gantry at a static z-position. In helical scanning protocols, the patient table moves in the z-direction simultaneously with rotation of the gantry. Thus, attenuation data is acquired for each of a series of different angular positions, $\phi$, at each of series of different axial positions, z.

Each emission and detection event at a particular [z, $\phi$] position may be referred to as a projection event' and the detection data acquired over the course of the emission and detection events of the scan is referred to as the projection data of the scan. The projection data can be converted into a series of axial images, each axial image representing an image of a slice through the imaged body across a plane parallel with the radial, r, and circumferential, $\varphi$, directions at a particular z position and with a view facing in the z-direction. The detection data acquired for a single projection comprises x-ray measurement data for each detector pixel of the detector. In some embodiments, the detector array may be a 2D array. For example, where the x-ray source 64 is a divergent beam source such as a cone beam source, a 2D detector may be used.

Fig. 4 schematically illustrates the projection data 72 acquired for a single projection. This comprises detector measurement data for each of a 2D array of pixels 74. The measurement data for the different pixels 74 are indexed by index, p. The detector data for each projection might be referred to a projection image, where each projection image comprises measurement data for each pixel 74 of the detector.

Thus, the projection data acquired in the CT scan includes one projection per [z, $\phi$] position, and one projection includes detection/measurement data for each detector pixel, p of a detector array. In other words, one projection includes pixel data for each detector pixel, p, for one [z, $\varphi$] position.

In some embodiments, the CT imaging apparatus is operable to acquire spectral CT projection data. In spectral CT imaging, measurements are acquired for each pixel of each projection for each of two or more different x-ray emission energies. For each pixel of each projection, the measurements for the different x-ray emission energies may be recorded in two or more different energy bins. The energy bins may be indexed by the index, n. Thus, each projection may include measurement data in each energy bin, n, for each detector pixel, p, for each [$\varphi$, z] position of the scanner. Thus, the projection data [$\varphi$, z, p, n] for the scan may include measurement data in each energy bin, n, for each detector pixel, p, for each [$\varphi$, z] position of the scanner. Furthermore, it is noted that each [$\varphi$, z] position of the scanner may be parameterized by a projection index, t.

As discussed above, the provided method is for off-focal correction of spectral x-ray data acquired using a spectral x-ray imaging apparatus. The off-focal correction is for reducing a contribution of off-focal radiation emitted by an x-ray source of the x-ray imaging apparatus.

Fig. 5 schematically illustrates an example x-ray source 64 emitting primary radiation 82 and off-focal radiation 84.

The x-ray source 64 for example comprises an x-ray tube which emits primary radiation 82 from a focal spot area 64a of the x-ray source. The x-ray source 64 further emits off-focal radiation 84 from a second area 64b of the x-ray source outside of the focal spot area 64a. For example, the second area may be an area surrounding or partially surrounding the focal spot area 64. Fig. 5 schematically illustrates how the x-ray detector 66 detects radiation contributions from both the primary 82 and the off-focal 84 radiation after the radiation passes through a body 92 being imaged, such as a patient.

As will be known by the skilled reader, the focal spot of an x-ray tube source is the area on the anode of the x-ray tube at which an electron beam from the cathode strikes. This interaction produces x-ray emissions. In particular, as electrons hit the focal spot, they decelerate causing conversion of their kinetic energy into x-rays. Off-focal radiation refers to x-ray radiation which is emitted from parts of the x-ray tube other than the intended focal spot. In particular, some electrons from the cathode may scatter and strike parts of the anode or the tube other than the intended focal spot, producing additional, unintended x-ray photons. In the context of this disclosure, the focal spot area 64a refers to the intended focal spot for the electrons striking the anode, while the second area 64b refers to collectively to areas which emit x-ray radiation but which are not part of the focal spot area 64a. The second area might otherwise be referred to as the off-focal area.

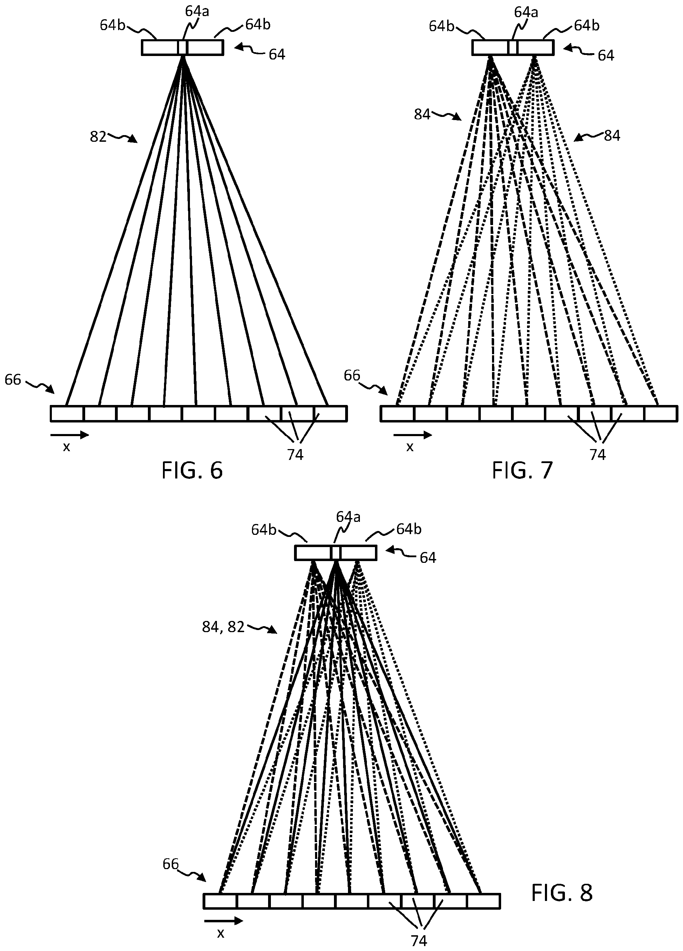

Fig. 6 and Fig. 7 show further schematic illustrations of emission of primary radiation 82 and off-focal radiation 84 respectively from an x-ray source 64 to pixels 74 of an x-ray detector 66. Just one row or column of pixels is shown in Figs. 6-8 for ease of illustration. The detector may be a one-dimensional detector which only has a single row/column of pixels or could be a 2D detector, and where Figs. 6-8 are showing just one of the rows or columns. The row or column of pixels in Figs. 6-8 is shown as extending in the x-direction, within the plane of rotation. It is across this direction that off-focal contributions are typically strongest. However, off-focal contributions along the z-direction could also be considered. See Fig. 3 for the alignments of the x, y and z directions relative to the scanner.

Fig. 6 schematically illustrates the receipt of primary radiation from the focal spot area 64a of an x-ray source at each pixel 74 of the detector array. Fig. 7 schematically illustrates the receipt of off-focal radiation from the second area 64b of the x-ray source 64 at each pixel 74 of the detector array. The schematic elevation view of Fig. 6 and Fig. 7 show only a single row of detector pixels 74, but it will be appreciated that the x-ray emissions would be received across all pixels of the detector array, including any pixels not visible in Fig. 6 in the case of a 2D detector array.

For completeness, Fig. 8 shows receipt of both the primary 82 and off-focal 84 radiation at each pixel 74 of the detector array 66 from the focal spot area 64a and the second area 64b respectively of the x-ray source 64.

As discussed above, embodiments of the invention are based on estimating the off-focal radiation contribution to the measurement data of each pixel of the detector of each projection, in each spectral channel. Example means for implementing this feature will now be discussed in more detail.

As discussed above, as a general proposition, it is proposed to estimate the off-focal radiation contribution based on applying a first material decomposition to the spectral measurement data and using the material decomposition information to infer an off-focal radiation contribution at each pixel.

This may be performed based on computing an estimated attenuation of the known off-focal radiation emission spectrum by the decomposed materials of the body along an estimated emission path from the off-focal area 64b of the x-ray source to the relevant pixel. The relevant pixel may be referred to as the pixel-to-be-corrected in some of the forthcoming discussion.

Conventional methods of off-focal radiation correction implicitly assume that the spectra of the off-focal radiation and of the primary radiation (the radiation emitted from the focal spot) are identical. However, considering that off-focal radiation is mainly generated by back-scattered electrons from the anode, it can be recognized that this assumption is not exact. Indeed, in literature in the field, it has been illustrated that off-focal radiation and primary radiation can have significantly different spectra. For example, reference is made to Fig. 8 of the paper: Ali ES, Rogers DW. Quantifying the effect of off-focal radiation on the output of kilovoltage x-ray systems. Med Phys. 2008 Sep;35(9):4149-60. doi: 10.1118/1.2966348. PMID: 18841868. This shows an illustration of primary radiation and off-focal radiation spectra. It can be seen that the off-focal radiation has a lower effective energy.

Embodiments of the present invention take into account the differing spectral composition of the primary radiation and off-focal radiation.

As discussed above, material decomposition is a method that converts the spectral measurement data into path lengths of different materials or physical attenuation mechanisms, e.g., photoelectric effect and Compton scattering. For the purposes of this disclosure, reference to 'material decomposition' and 'materials' includes reference to decomposition into basis attenuation phenomena such as Compton scatter and photoelectric effect.

The output of the material decomposition is called material measurement data or material projection data in the following.

As discussed above, different methods are possible for performing material decomposition, e.g., based on a look-up table, a maximum likelihood approach, a polynomial model, an AI-based model, and others.

In a lookup table approach, a lookup table can be prepared in advance through known calibration methods which allows for mapping each set of multi-energy detection values for each given pixel (or equivalent attenuation values) of the detector to an equivalent set of material path lengths for a set of two or more basis materials or basis attenuation phenomena, e.g. Compton scatter and photoelectric effect. In other words, if a given pixel, p, yields an energy or attenuation measurement set $I(p) = [I_{E1}(p), I_{E2}(p)]$ for each of two energy levels $E_1$ and $E_2$, a lookup table can be prepared which permits mapping this pair of measurement values to a corresponding set of basis material path lengths for a pair of materials M1 and M2, $l(p) = [l_{M1}(p), l_{M2}(p)]$, for example , $l(p) = [l_{ph}(p), l_{cs}(p)]$, where the bases are the photoelectric effect and Compton scatter, i.e.

$$\mathbf{I}(p) = [I_{E1}(p), I_{E2}(p)] \implies l(p) = [l_{M1}(p), l_{M2}(p)]$$

where **I**(p) is a vector of the energy or attenuation measurements in each of the n energy bins, $E_n$, of the pixel, *p,* of the detector and *l*(p) is a vector of the basis material path lengths for each of material M1 and M2.

Although this example shows only two energy bins and two materials in the decomposition, measurements for more than two energy bins can be acquired which enables determining material path lengths for more than two different materials.

Instead of a lookup table, various other methods for performing the mapping exist, as will be known by the skilled person.

The inference of the off-focal radiation contribution is based on modelling the detected x-ray energy in each energy bin, $E_n$, at each detector pixel, *p,* of each projection as a sum of a primary radiation contribution and an off-focal radiation contribution. This might be expressed as follows:

$$I^{meas}(E_n) = I_0^{prim}(E_n) \exp\left(-\mu_{M1} l_{M1}^{prim} - \mu_{M2} l_{M2}^{prim}\right) + I_0^{off}(E_n) \exp\left(-\mu_{M1} l_{M1}^{off} - \mu_{M2} l_{M2}^{off}\right)$$

Here,

$I^{meas}(E_n)$ is the total/overall measured intensity of energy component $E_n$ of the x-ray beam at a detector pixel after it has passed through the body, i.e. the measured x-ray energy value at a pixel in energy bin $E_n$. $I_0^{prim}$ represents the initial intensity of the $E_n$ component of the primary ('prim') radiation 82 at the x-ray source before it enters the material.

$\mu_{M1}$ is the linear attenuation coefficient for a first basis material or attenuation phenomenon, M1, at energy $E_n$. This quantifies how much M1 contributes to the attenuation of an X-ray beam having energy $E_n$. By way of example, M1 might represent the photoelectric effect.

$\mu_{M2}$ is the linear attenuation coefficient for a second basis material or attenuation phenomenon, M2, at energy $E_n$. This quantifies how much M2 contributes to the attenuation of an X-ray beam having energy $E_n$. For example, M2 might represent Compton Scatter.

$l_{M1}^{prim}$ is the effective material path length of the body from the focal spot area 64a of the x-ray source 64 to the detector 66 over which M1 is the dominant attenuator of x-rays.

$l_{M2}^{prim}$ is the effective material path length of the body from the focal spot area 64a of the x-ray source 64 to the detector 66 over which M2 is the dominant attenuator of x-rays.

$I_0^{off}$ represents the initial intensity of the $E_n$ component of the off-focal radiation 84 at the x-ray source before it enters the material.

$l_{M1}^{off}$ is the effective material path length of the body from the second area 64b of the x-ray source 64 to the detector 66 over which M1 is the dominant attenuator of x-rays.

$l_{M2}^{off}$ is the effective material path length of the body from the second area 64b of the x-ray source 64 to the detector 66 over which M2 is the dominant attenuator of x-rays.

It is possible to generalize this further to cover a case in which there are multiple sources of off-focal radiation, indexed by the index j as follows:

$$I^{meas}(E_n) = I_0^{prim}(E_n) \exp\left(-\mu_{M1} l_{M1}^{prim} - \mu_{M2} l_{M2}^{prim}\right)$$
$$+ \sum_j I_0^{off,j}(E_n) \exp\left(-\mu_{M1} l_{M1}^{off,j} - \mu_{M2} l_{M2}^{off,j}\right)$$

Although reference has been made to a material decomposition into Compton scatter and photoelectric effect attenuation contributions, any other choice of decomposition bases M1, M2 can be chosen, e.g. different effects or different materials, e.g. bone and soft tissue, iodine and soft tissue, calcium and soft tissue, fat and water, air and soft tissue, and so on. Alternative decompositions can be achieved simply by replacing the attenuation coefficients, $\mu$, for the materials or effects with the attenuation coefficients of the target basis materials or effects at the relevant measured energy levels, $E_n$.

The method for determining the off-focal radiation contributions comprises a step of performing a first material decomposition using the original multi-energy detector measurement data for each pixel, p, and each projection, t:

$$\mathbf{I}^{meas}(p, t) = [I_{E1}(p, t), I_{E2}(p, t), \ldots, I_{En}(p, t)].$$

The first material decomposition yields values for the material path lengths $l_{Mn}$ for each basis material or effect, Mn, and for each pixel, p, and each x-ray projection, where the x-ray projections may be indexed by the index, t:

$$\boldsymbol{l}(p, t) = [l_{M1}(p, t), l_{M2}(p, t), \ldots, l_{Mn}(p, t)]$$

for basis material M1 to Mn, for each pixel $p$ of each x-ray projection, $t$. This data may be referred to as the material measurement data for the pixels, p.

For a given pixel, $p$, of a given projection, t, it is necessary to determine the off-focal material path lengths $l_{M1}^{off,j}(p, t)$ and $l_{M2}^{off,j}(p, t)$.

For a given pixel-to-be-corrected, it is proposed in accordance with at least some embodiments of the invention to use the material path lengths $\boldsymbol{l}(p, t)$ of at least one different pixel, p, of a same projection, t, or to use the material path lengths $\boldsymbol{l}(p, t)$ of a same or different pixel of a different projection, t, as an approximation for the off-focal material path lengths.

In other words, it is proposed to make an assumption or approximation that that $l_{M1}^{off,j}(p, t)$ is equal to $l_{M1}(p + x, t + y)$ and that $l_{M2}^{off,j}(p, t)$ is equal to $l_{sc}(p + x, t + y)$. The values of x and y may be pre-determined, e.g. 1 or -1, or may be computed on the fly. This process is described in more detail later.

In other words, it corresponds to a pixel within a neighborhood of the operative pixel. In other words, an assumption is made that the off-focal material path lengths for a given pixel, $p_a$, are equal to the primary radiation material path lengths of another pixel, $p_b$, e.g. a neighboring pixel of a same projection or a same or different pixel position of a different projection, or equal to a (possibly weighted) sum of the primary radiation material path lengths of a plurality of other pixels.

Although of course the material path lengths computed for the nearby pixels will be affected by their own off-focal contributions, the dominant contribution will always be from the primary radiation. Thus, for present purposes of providing an estimate of the material path lengths along the path from the off-focal area to the pixel-to-be-corrected, these are sufficient.

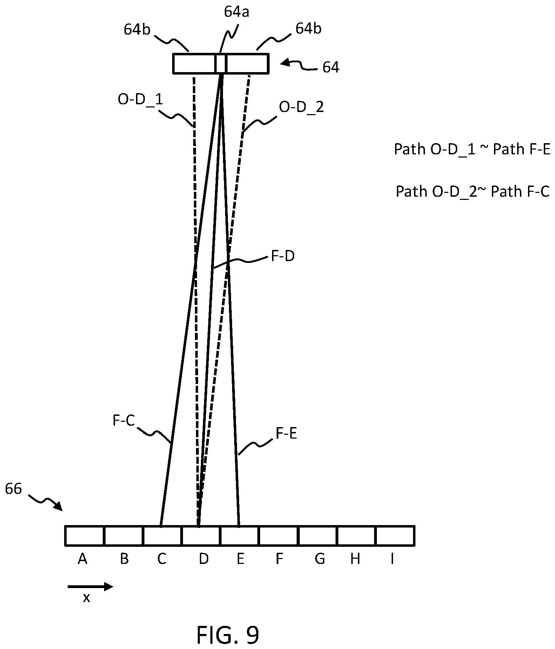

This approximation can be understood from the schematic illustration of Fig. 9.

Considering by way of illustration pixel D in Fig. 9, where pixel D is a pixel-to-be-corrected. Fig. 9 schematically illustrates pixel D receiving a primary x-ray radiation contribution, F-D, from the focal spot area 64a of the x-ray source, and off-focal radiation contributions, O-D_1 and O-D_2 from the second area 64b of the x-ray source 64. For off-focal contribution O-D_1, it can be seen that the physical path through the imaged body from the x-ray source to the detector has a very similar trajectory direction/angle as primary radiation path, F-E, from the focal spot area 64a of the source to pixel E of the detector. Likewise, for off-focal contribution O-D_2, it can be seen that the physical path through the imaged body from the x-ray source to the detector has a very similar trajectory direction/angle as primary radiation path, F-C, from the focal spot area 64a of the source to pixel C of the detector. It will be appreciated that in the context of a real-world imaging system, the distance between the source 64 and the detector 66 is very much greater than the distance between neighboring pixels of the detector, such that, in a real-world system, trajectory O-D_1 and F-E are, to a first approximation, almost coincident spatially. Likewise for trajectory O-D_2 and F_C.

Thus it can be seen that the off-focal contribution to the overall material path length for material M1 at pixel D can be estimated as approximately equal to the material path length for material M1 at pixel C plus the material path length for material M1 at pixel E.

In this way, for a given pixel, $p$ of a given projection, t, an approximation can be made of off-focal material paths lengths $l_{M1}^{off,j}$ and $l_{M2}^{off,j}$ based on the primary material path lengths of one or more different pixels of the same projection or a different projection.

Using these approximations, a radiation contribution at the pixel p by the off-focal radiation in energy bin $E_n$ can be estimated by evaluating the following:

$$I^{off}(p, t, E_n) = \sum_j I_0^{off,j}(E_n) \exp\left(-\mu_{M1} l_{M1}^{off,j}(p, t) - \mu_{M2} l_{M2}^{off,j}(p, t)\right)$$

where

j indexes the different sources of off-focal radiation,

$I^{off,j}(p, E_n)$ is the total/overall off-focal contribution of off-focal source $j$ to the measured energy component $E_n$ of the x-ray beam at a detector pixel, $p$, for projection t, after it has passed through the body, i.e. the measured x-ray energy value at a pixel in energy bin $E_n$.

$\mu_{M1}$ is the linear attenuation coefficient for a first basis material or attenuation phenomenon, M1, at energy $E_n$. This quantifies how much M1 contributes to the attenuation of an x-ray beam having energy $E_n$. By way of example, M1 might represent the photoelectric effect.

$\mu_{M2}$ is the linear attenuation coefficient for a second basis material or attenuation phenomenon, M2, at energy $E_n$. This quantifies how much M2 contributes to the attenuation of an x-ray beam having energy $E_n$. For example, M2 might represent Compton Scatter.

$I_0^{off,j}(E_n)$ represents the initial intensity of the $E_n$ component of the off-focal radiation source $j$ at the x-ray source before it enters the material.

$l_{M1}^{off,j}(p, t)$ is the effective material path length, for off-focal radiation source j, through the body from the second area 64b of the x-ray source 64 to the detector pixel, $p$, in projection t, over which M1 is the dominant attenuator of x-rays.

$l_{M2}^{off,j}(p, t)$ is the effective material path length, for off-focal radiation source j, through the body from the second area 64b of the x-ray source 64 to the detector pixel, $p$, in projection, $t$, over which M2 is the dominant attenuator of x-rays.

This approximation of the off-focal radiation contribution to pixel $p$ in each energy bin $E_n$ can then be subtracted from the original measured energy at pixel p, $I^{meas}(p, t, E_n)$ to achieve a corrected value, $I^{corr}(p, t, E_n)$, for the measurement data:

$$I^{corr}(p, t, E_n) = I^{meas}(p, t, E_n) - I^{off}(p, t, E_n)$$

It is noted that the above equation for $I^{off}(p, t, E_n)$ does not fully take account of detection sensitivity effects of detector pixels. A more comprehensive version of this equation can be found in the paper: Roessl E, et al. Sensitivity of photon-counting based K-edge imaging in X-ray computed tomography. IEEE Trans Med Imaging. 2011 Sep;30(9): 1678-90. doi: 10.1109/TMI.2011.2142188. In this paper, equation (3) for example provides a more correct description of the signal induced in a photon counting detector, including factoring in a sensitivity function for each energy bin of the detector.

In this regard, it is also noted that more generally, the equations set out above may be generalized to refer to any forward model including one which takes into account further factors in addition to the material attenuation effect which is modelled by the exponential decay functions in the expressions above. For example, the expression set out above for the total radiation measured at a given pixel of a given projection, $I^{meas}(E_n)$, could be generalized as:

$$I^{meas}(E_n) = I^{prim}(E_n) + \sum_j I^{off,j}(E_n)$$

$$= FWD\left(I_0^{prim}(E_n), l_{M1}^{prim}, l_{M2}^{prim}\right) + \sum_j k_j \cdot FWD(I_0^{off,j}(E_n), l_{M1}^{off,j}, l_{M2}^{off,j})$$

Here, FWD represents the spectral forward model,

$I_0^{prim}$ represents the initial intensity of the $E_n$ component of the primary radiation 82 at the x-ray source before it enters the material.

$I_0^{off,j}(E_n)$ represents the initial intensity of the $E_n$ component of the off-focal radiation source $j$ at the x-ray source before it enters the material.

$l_{M1}^{off,j}$ is the effective material path length, for off-focal radiation source j, through the body from the second area 64b of the x-ray source 64 to the detector pixel over which M1 is the dominant attenuator of x-rays.

$l_{M2}^{off,j}$ is the effective material path length, for off-focal radiation source $j$, through the body from the second area 64b of the x-ray source 64 to the detector pixel over which M2 is the dominant attenuator of x-rays.

$l_{M1}^{prim}$ is the effective material path length of the body from the focal spot area 64a of the x-ray source 64 to the detector 66 over which M1 is the dominant attenuator of x-rays.

$l_{M2}^{prim}$ is the effective material path length of the body from the focal spot area 64a of the x-ray source 64 to the detector 66 over which M2 is the dominant attenuator of x-rays.

$k_j$ are a set of weighting coefficients to be applied to the different sources of off-focal radiation. The weightings can be determined in advance and retrieved from a memory or register for example.

With regards to determining which nearby pixels' material measurement data to use to inform the estimate of the off-focal contribution (i.e. which nearby pixels' material path lengths to use), in some embodiments, this may be done in a very simple approximate way by choosing to use the material data for a nearest neighboring pixel or pixels, or a defined number of nearest neighboring pixels, possibly these being combined in a weighted combination. The weightings may be pre-defined in advance in the latter case.

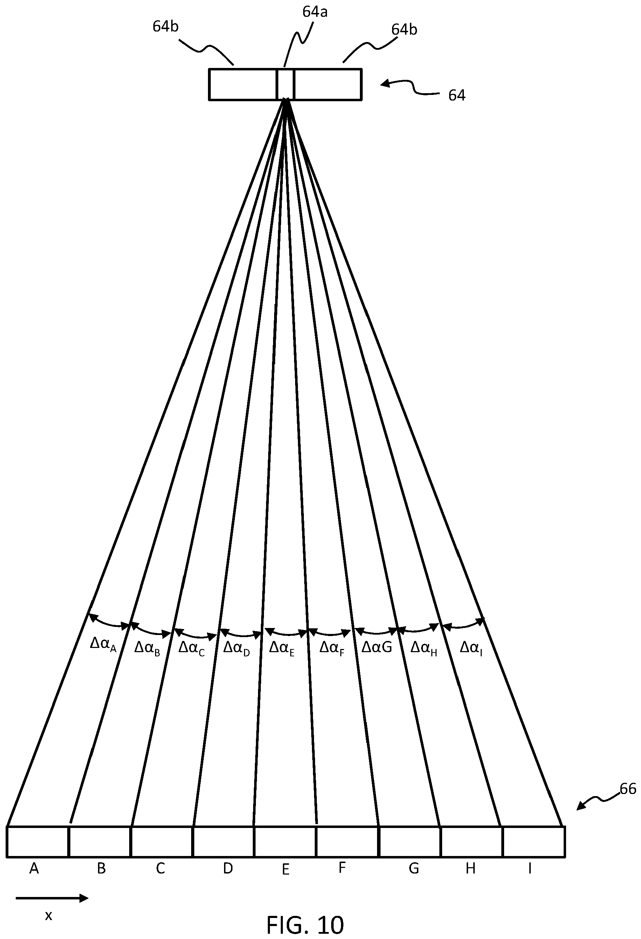

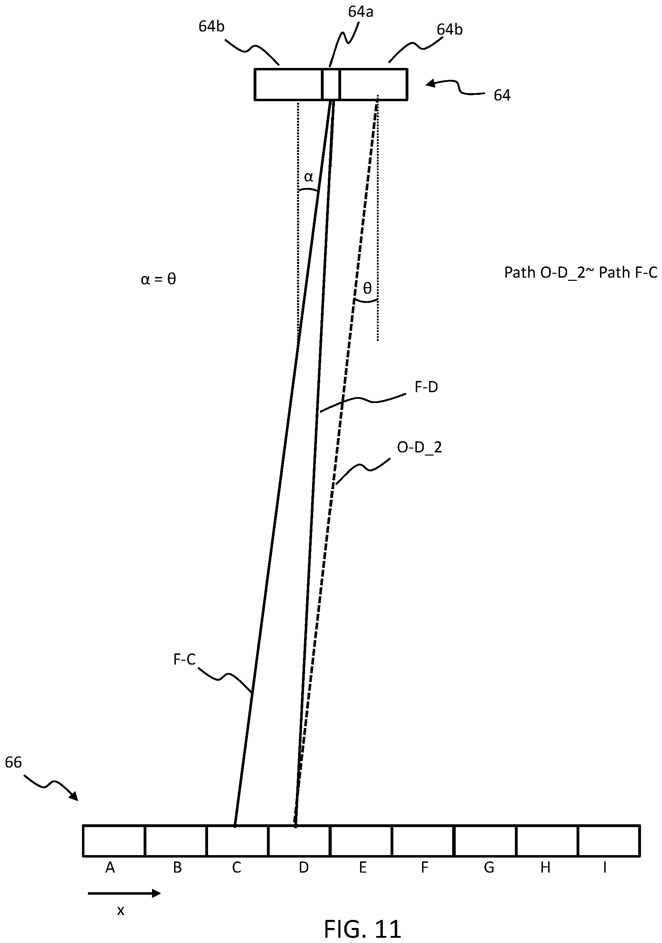

Additionally or alternatively, a geometric approach might be applied to explicitly determine the particular one or more other pixels of the detector during the same or different projections whose material data is to be used to estimate the off-focal contribution. The principles underlying this approach will now be briefly described with reference to Fig. 10 and Fig. 11.

Fig. 10 illustrates again a schematic representation of pixels A to I along one dimension of a detector array 66 and an x-ray source having a focal spot area 64a and a second, off-focal area 64b. For present purposes of illustration, the x-ray radiation emitted from just the focal spot area 64a is shown. This is modelled as fanning out from the focal spot area 64a across an angular range that spans all of the detector pixels A to I of the detector along the illustrated dimension, x. Each detector pixel can accordingly be modelled as receiving from the focal spot area the radiation emitted from the focal spot area 64a of the x-ray source across a respective angular range, $\Delta\alpha_m$, of the radiation projection, where m is an index of detector pixel, and where $\Delta\alpha_m$ represents the range of projection angles (defined in this case relative to the emission point of the focal spot 64a) across which the detector pixel m receives radiation from the focal spot.

The set of angle ranges $\Delta\alpha_m$, for each pixel of a given detector can be determined in advance for a given detector-source setup, since the relative position of the source and detector remains static for a given scanner setup. Thus this information can be determined in advance empirically or through simple geometric methods and stored as reference information in a memory.

This information can be used to identify in a relatively simple way the best set of one or more further detector pixels whose material measurement data should be used to estimate the off-focal contribution for a given pixel whose data is to be corrected. In particular, this can be done by first identifying the angle at which off-focal radiation is received at the given pixel to-be-corrected from the or each off-focal emission area of the x-ray source and then simply looking up the detector pixel whose respective angular range of received primary radiation includes the said determined angle.

This is illustrated schematically in Fig. 11 which shows on-focal projection rays F-D and F-C emitted from the focal spot area 64a to, respectively, pixel D and pixel C during the same projection. Fig. 11 further illustrates an off-focal ray O-D_2 emitted by second off-focal area 64b of the source 64. For ease of illustration, just the radiation emitted from the second off-focal area 64b is shown in Fig. 11. The emission angle of the off-focal ray O-D_2 from the off-focal source relative to a normal axis of the source is shown as angle $\theta$. Fig. 11 illustrates an on-focal (primary radiation) ray F-C emitted from the focal source 64a toward the detector at angle $\alpha$ in the particular case where $\alpha = \theta$. In the scenario illustrated, a ray emitted from the focal spot area 64a at angle $\alpha=\theta$ falls incident upon detector pixel C, i.e. falls within the angle range $\Delta\alpha_c$. This could be looked up from a pre-stored reference table for example. Accordingly, in this case, the material decomposition data for pixel C would be used to determine the estimated off-focal contribution to the measurement data of pixel D from the second off-focal source area 64b.

A more sophisticated treatment of an example process for selecting the material decomposition data from other pixels of the same or different projections which is to be used to estimate the off-focal contribution for a pixel-to-be-corrected is described in detail in the document US6628744B1, with particular reference to the description between column 4, line 21 and column 7, line 6. The off-focal correction method described in US6628744B1 is different to that of the present invention in that it directly uses measurement data of other pixels in the same or other projections to correct for the off-focal contribution to a given pixel. By contrast, as has been explained, embodiments of the present invention use first-pass material decomposition data pertaining to other pixels of the same or different projections to infer an off-focal contribution. However, the method described for identifying the most relevant other pixels whose data is to be used can be applied mutatis mutandis to embodiments of the present invention. In particular, the geometrical considerations are the same. Once the relevant set of one or more other pixels whose data is to be used are found, the method can proceed in accordance with the remaining steps and processes as described in this document.

The approach in US6628744B1 may provide more accurate results than the approach described above with reference to Fig. 10 and Fig. 11 since it proposes use of material measurement data acquired for detector pixels in not just the same projection but other projections. This approach allows for identifying data corresponding to a beam path trajectory in the acquired material measurement data which more exactly aligns with the spatial trajectory of the expected beam path of the off-focal radiation for the given pixel whose data is being corrected.

Optionally, the method may further comprise performing a further material decomposition procedure by applying a material decomposition algorithm to the corrected measurement data, $I^{corr}$. This may be the same material decomposition algorithm used to generate the first set of material decomposition data.

Optionally, the correction procedure outlined above may be repeated for one or more further iterations to further improve the off-focal correction. In particular, the method may comprise: performing a further material decomposition procedure by applying a material decomposition algorithm to the corrected measurement data, $I^{corr}$ to generate further material measurement data, and using the further material measurement data as input the previously described step of determining the off-focal radiation contribution to the measurement data of each pixel.

Optionally, the method may further comprise an image reconstruction operation applied to the output of the further material decomposition. This allows for one or more material images to be generated, each corresponding to a different material, and representing a concentration of the given material at each voxel/pixel location in the image. Each image for a given material is known as a material image or material decomposition image.

For example, for a material decomposition image for material M1, the intensity of each pixel is proportional to the effective/equivalent concentration of material M1 along an x-ray path that corresponds to that pixel. Specifically, it reflects the amount of x-ray attenuation attributed to material M1, which is a function of both the density (mass attenuation coefficient) of material M1 and its volume (the length of the path the x-ray travels through it) within the voxel (three-dimensional pixel) represented by that pixel in the image.

Thus, by way of summary, the basic processing approach proposed in accordance with embodiments of the invention may be summarized as follows:

A first material decomposition is applied which either does not include any dedicated treatment for off-focal radiation, or includes a conventional off-focal correction.

The result of this first material decomposition is a set of material projection data which permits calculation for each detector pixel an approximation of the contribution of the off-focal radiation to the spectral measurements of this pixel. This can be achieved by attenuating the x-ray spectrum of the off-focal radiation by the absorption spectra of the materials determined for neighboring pixels or by the absorption spectra of the same pixel under different gantry angles and z-positions, and converting to signal contributions of the spectral measurements utilizing a spectral detector model.

Correction of the original measurement data is performed using the determined off-focal contributions. One simple method for achieving this is to subtract these off-focal contributions from the original spectral measurement data.

Finally, a second material decomposition may be executed based on the corrected spectral measurements. Here, only the primary radiation spectrum is used in the spectral model of the decomposition.

If the resulting material projection data of step 4 is accurate enough, material images can be reconstructed. Otherwise, the material projection data of step 4 may be used as input for step 2, and step 2-4 are repeated once or several times.

The material decomposition methods applied in step 1 and 4 may be the same, or may be different (e.g., method in step 1 may be a faster method, but less accurate).

In the context of CT imaging, the method of off-focal correction described in this disclosure may be performed at a same point along the CT data pre-processing pipeline as for conventional off-focal correction.

In some embodiments, a scatter correction procedure may additionally be performed. Preferably this is a spectral scatter correction, i.e. an energy-specific scatter correction. This may be performed prior to the off-focal correction, or may be performed after the material decomposition of step 1 mentioned above, in parallel with the off-focal correction.

It is noted that although descriptions presented above were illustrated with reference to schematic drawings showing a detector with detector pixels extending along one dimension (namely, the dimension in the plane of rotation of the detector/scanner pair, i.e. a dimension perpendicular to the z-axis), a real-world detector may comprise a two dimensional detector pixel array. In this case, the above methods can be readily applied to a detector pixel set extending in the two dimensions. However, with regards to determining an off-focal correction for any given pixel, here an acceptable and accurate estimation is to just take into account off-focal contributions along the dimension in the plane of rotation. This one-dimensional approximation is a valid approach, since, due to the small anode angle, the x-ray radiation projection from the source (both the focal spot area and the off-focal areas) is minimal in the z-direction, and mostly in the direction of the plane of rotation. As a result, the off-focal radiation is effectively emitted from a line in the plane of rotation.

A variation on the above-described embodiments may be considered in which the first-pass material decomposition data is first reconstructed to form an image, and then the reconstructed image is used to determine the estimated off-focal contributions to the measurement data of each detector pixel of each projection.

For example, according to some embodiments, the method may be varied in accordance with the following summary.

A first step is to perform the first pass material decomposition as is performed in the other described embodiments outlined above.

Following this, the material decomposition data is reconstructed to form a set of one or more material images.

Following this, a direct spatial path can be identified through the reconstructed image data from the off-focal area of the x-ray source to each detector pixel of each projection and then the forward model applied to model the expected radiation contribution received from the off-focal area at each given pixel if travelling along that material path.

The advantage of this approach is that material paths for arbitrary source points near the focal spot could be calculated more readily, at the cost of some additional computational effort of reconstruction and forward projection.

Embodiments of the invention described above employ a processing device. The processing device may in general comprise a single processor or a plurality of processors. It may be located in a single containing device, structure or unit, or it may be distributed between a plurality of different devices, structures or units. Reference therefore to the processing device being adapted or configured to perform a particular step or task may correspond to that step or task being performed by any one or more of a plurality of processing components, either alone or in combination. The skilled person will understand how such a distributed processing device can be implemented. The processing device includes a communication module or input/output for receiving data and outputting data to further components.

The one or more processors of the processing device can be implemented in numerous ways, with software and/or hardware, to perform the various functions required. A processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

**Claims**

**1.** A method for off-focal correction of spectral x-ray data acquired using a spectral x-ray imaging apparatus comprising an x-ray source which emits primary radiation from a focal spot area of the x-ray source and off-focal radiation from a second area of the x-ray source outside of the focal spot area, the method comprising:

> receiving spectral x-ray measurement data comprising measurement data for each of a plurality of pixels of an x-ray detector in each of two or more energy channels for at least one x-ray projection;
> applying a material decomposition algorithm to convert the spectral measurement data into material measurement data, where the material measurement data comprises, for each pixel of the detector, a respective attenuation contribution of each of two or more materials along a beam path from the x-ray source to the detector pixel;
> obtaining an indication of an x-ray absorption spectrum for each of the two or more materials;
> obtaining an indication of an x-ray emission spectrum for the off-focal radiation emitted by the x-ray source;
> for each pixel of the measurement data of the at least one projection, using the material measurement data to compute an estimated attenuation of the said off-focal radiation, having the said emission spectrum, by the said two or more materials, having the said x-ray absorption spectra, along an expected beam path from the second area of the x-ray source to the detector pixel to thereby compute an estimated off-focal radiation contribution to the spectral x-ray measurement data of the pixel, in each spectral channel;
> correcting the received spectral measurement data to remove or reduce the estimated off-focal contribution for each spectral channel for each pixel of the measurement data to yield corrected spectral x-ray measurement data.

2. The method of claim 1, further comprising generating a data output representative of the corrected spectral measurement data.

3. The method of claim 1 or 2, wherein the method further comprises re-computing a material decomposition using the corrected spectral measurement data.

4. The method of any preceding claim, wherein the step of determining the respective attenuation contribution of each of the two or more materials along a beam path from the second area of the source to the detector pixel comprises: determining, from the material measurement data, an estimated material path length of each of the two or more materials along the expected beam path from the second area of the source to the detector pixel.

5. The method of claim 4, wherein the step of computing an estimated attenuation of the off-focal emission spectrum along an expected beam path from the second area of the x-ray source to a detector pixel further comprises: computing an estimated attenuation of the off-focal radiation along said determined material path lengths.

6. The method of claim 5, wherein the step of determining the material path lengths along the expected beam path comprises identifying in the material measurement data computed material path lengths computed for a beam path:

> from the focal spot area of the x-ray source to a different detector pixel of a same x-ray projection; and
> having a path trajectory which is within a threshold degree of alignment spatially with said expected beam path.

7. The method of claim 5, wherein the step of determining the material path lengths along the expected beam path comprises identifying in the material measurement data computed material path lengths computed for a beam path:

> from the focal spot area of the x-ray source to a same or different detector pixel of a different x-ray projection; and
> having a path trajectory which is within a threshold degree of alignment spatially with said expected beam path.

8. The method of any preceding claim, wherein the x-ray imaging apparatus is a computed tomography (CT) imaging apparatus, and wherein the x-ray measurement data is CT projection data acquired by the CT imaging apparatus.

9. A computer program product comprising computer program code configured, when run on a processor, to cause the processor to perform a method in accordance with any preceding claim.

10. A processing device comprising one or more processors configured to perform a method in accordance with any of claims 1-9.

11. A system, comprising:

a processing device in accordance with claim 10; and
an x-ray imaging apparatus operatively coupled with the processing device.

12. A system in accordance with claim 11, wherein the x-ray imaging apparatus is a computed tomography (CT) imaging apparatus operatively coupled with the processing device.

FIG. 1

FIG. 2

FIG. 3

FIG. 4

FIG. 5

FIG. 6

FIG. 7

FIG. 8

Path O-D_1 ~ Path F-E

Path O-D_2~ Path F-C

FIG. 9

FIG. 10

Path O-D_2~ Path F-C

$\alpha = \theta$

**FIG. 11**

# EUROPEAN SEARCH REPORT

**Application Number**

EP 24 18 9244

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2013/156163 A1 (LIU XIN [US] ET AL) 20 June 2013 (2013-06-20) * abstract, claims 1-23, [0001-0078], Figs. 1-9 * | 1-12 | INV. G06T11/00 ADD. A61B6/00 |
| A | EP 3 992 912 A1 (KONINKLIJKE PHILIPS NV [NL]) 4 May 2022 (2022-05-04) * abstract, claims 1-15, [0001-0072], Figs. 1-4 * | 1-12 | |

**TECHNICAL FIELDS SEARCHED (IPC)**

G06T
A61B

The present search report has been drawn up for all claims

| Place of search | Date of completion of the search | Examiner |
|---|---|---|
| Munich | 14 January 2025 | Borotschnig, Hermann |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 24 18 9244

This annex lists the patent family members relating to the patent documents cited in the above-mentioned European search report.
The members are as contained in the European Patent Office EDP file on
The European Patent Office is in no way liable for these particulars which are merely given for the purpose of information.

14-01-2025

| Patent document cited in search report | Publication date | Patent family member(s) | Publication date |
|---|---|---|---|
| US 2013156163 A1 | 20-06-2013 | NONE | |
| EP 3992912 A1 | 04-05-2022 | EP 3992912 A1 | 04-05-2022 |
| | | WO 2022096401 A1 | 12-05-2022 |

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6628744 B1

**Non-patent literature cited in the description**

- **K. C. ZIMMERMAN** ; **T. G. SCHMIDT**. Experimental comparison of empirical material decomposition methods for spectral CT. *Physics in Medicine & Biology*, 2015, vol. 60, 3175-3191
- **ALI ES** ; **ROGERS DW**. Quantifying the effect of off-focal radiation on the output of kilovoltage x-ray systems.. *Med Phys.*, September 2008, vol. 35 (9), 4149-60

- **ROESSL E et al.** Sensitivity of photon-counting based K-edge imaging in X-ray computed tomography.. *IEEE Trans Med Imaging.*, September 2011, vol. 30 (9), 1678-90